# EUROPEAN PATENT APPLICATION

(11) **EP 4 278 952 A1**
(43) Date of publication of application: **22.11.2023**
(21) Application number: 22174067.3
(22) Date of filing: 18.05.2022
(51) Int. Cl.: A61B 5/00, A61B 5/1455

(54) **OPTICAL SENSOR AND METHOD FOR FABRICATING THE SAME**

(71) Applicant: Universität Zürich, 8006 Zürich (CH)
(72) Inventor: NÜSSLI, Anais, 9500 Will (CH); KLEISER, Stefan, 79639 Grenzach-Wyhlen (DE); OSTOJIC, Daniel, 8046 Zürich (CH); KALYANOV, Alexander, 5507 Mellingen (CH)
(74) Representative: Mertzlufft-Paufler, Cornelius

(57) **Abstract**

For improving the conformability of a head (2) of an optical sensor (1) and also shielding from ambient light, and for avoiding pressure marks on skin, a novel design of an optical sensor (1) is suggested which features a signal carrier (27), for example in the form of a flexible PCB (8) or in the form of a bundle of light guides (29), which are formed into a smooth curve (20) by a supporting structure (33) that preferably features at least two pillars (11). The support structure, which may still be bendable and allow deformation of the smooth curve (20), helps in guiding the signal carrier (27) in a way that avoids the formation of pressure spots and helps to bring a lower contact surface (12) of the sensor's head (2) into close vicinity to the skin during a measurement

## Description

The present disclosure concerns an optical sensor and a method for fabricating the same using a molding step.

The sensor may be configured as a NIRS-sensor or as an oximeter, for example, such that it can be used for measuring a physiological parameter in human tissue. The sensor comprises a sensor head bearing at least one light source and at least one detector which are arranged on a carrier substrate. The sensor head may define a contact plane to be brought into skin contact during a measurement. Finally, the sensor features a cable comprising at least one signal carrier that is configured to route signals to and/or from the sensor head, for example to and/or from an external electronic or optical unit.

Such sensors are already used in clinical trials for measuring blood oxygenation in cerebral tissue of neonates. In such an application case, the sensor head must be brought in close vicinity to the curved shape of the head of the neonate. Any gap between a lower contact surface of the sensor head and the skull of the neonate must be avoided because this would allow ambient light to reach the measurement spot, which would deteriorate the measurement.

At the same time, the cable is necessary for a reliable measurement. The cable however, if formed too stiff, may get in conflict with the aim of attaching the sensor head in close skin contact, as the cable can limit the amount of bending that is achievable with the flexible sensor head.

Starting out from this background, the invention aims at providing a simple fabrication method and sensor design, which alleviates the afore-mentioned problem.

In accordance with the present invention, an optical sensor is provided according to claim 1, which solves the afore-mentioned problem. In particular, the invention proposes an optical sensor as introduced at the beginning, which, in addition, is characterized in that the carrier substrate is elevated above the contact plane by a distance D1.

Most preferably a middle portion of the at least one signal carrier, being located between the carrier substrate and a proximal portion of the at least one signal carrier, may follow a smooth curve. "Smooth" may be understood here, in particular, in that the curve shows no sharp kinks. Instead, the smooth curve shows a continuous course.

According to one possible embodiment, which is advantageous if the cable runs roughly parallel to the contact plane, the curve may show an S-shape. In other words, the middle portion may follow a smooth S-shaped curve.

Due to the smooth, in particular S-shaped, curve, the middle portion can thus bend downwards (along the z-axis) from the elevated level in which the carrier substrate is located to a lower level in which said contact plane is located. In other words, the at least one signal carrier may be designed as a flexible component, such that it offers no shape stability (other than for example a stiff PCB or a metal member).

As a major benefit of this approach, during an optical measurement, when the sensor head is attached onto the skull of a neonate, the portion of the signal carrier (which may be implemented as a flex-PCB for example) that extends into the cable can exit the head of the sensor as close to the patient's skin and as parallel thereto as possible. This avoids the formation of pressure marks on the delicate skin and also results in an optical shielding, thus preventing ambient light from penetrating into the region that is optically inspected with the sensor head. Accordingly, the measurement accuracy and reliability are improved as well as the ease of use of the sensor.

In addition, the proposed design of the sensor can allow, in particular, that a lower contact surface (defining said contact plane) of the sensor head and a lower surface of said cable can be arranged coplanar; i.e., the transition of the cable into the sensor head can be arranged completely flat / without a step, at least on the lower side that is facing the skin. Such a design offers significant advantages for the end user because pressure marks can be avoided on the skin, when the sensor head is brought into skin contact during a measurement. Moreover, the sensor can be brought much closer into contact, as compared to state-of-the-art designs.

For the same reasons, it is of advantage if the cable has a non-circular (e.g., approximately rectangular) cross-section, preferably with an aspect ratio (AR) of
AR = width/thickness > 2:1, preferably with AR > 5:1.

We note at this point already, that the signals mentioned above, which are routed by said signal carrier(s), may be electrical currents/voltages and/or optical signals, depending on the sensor design / implementation; the invention is applicable in all such cases.

According to the invention, there exist further advantageous embodiments solving the afore mentioned problems, which are described in the sub-claims and in the following, and which may be also used in combination, to solve the afore-mentioned problem most effectively.

The optical sensor can be applied most conveniently on the small, strongly curved heads of neonates if the sensor head is designed such that the sensor head can be bent (repeatedly and without destruction) in a section corresponding to the middle portion of the at least one signal carrier. Due to the flexible nature and bending of the sensor head, the course of the smooth curve of the at least one signal carrier (which itself is preferably flexible) may thus be reversibly changed during use of the optical sensor (i.e., the smooth curve/the at least one signal carrier may thus be flexed and/or bent itself during use of the optical sensor). Such a design can be obtained most simply, if the sensor head is formed from a flexible material, for example silicone and also the at least one signal carrier is formed from a flexible material (e.g., the at least one signal carrier may be at least one flexible light guide or it may be formed by a flexible printed circuit board, as will now be detailed).

For example, one embodiment suggests that the at least one signal carrier is at least one electrical conductor. This at least one electrical conductor may be preferably formed by a flexible printed circuit board. Most preferably, the mentioned carrier substrate can be arranged on or formed by the flexible printed circuit board (i.e., the carrier substrate and the flex-PCB may thus be designed as one piece / formed as one single member, in particular; for example, the flex-PCB may be stiffened with additional FR4-layers at the distal end to form the carrier substrate in this area). In such a case, the at least one light source and/or said at least one detector may be optoelectronic components, which are mounted on the carrier substrate. Such a design is highly beneficial for achieving a high flexibility of the sensor head in the section corresponding to the middle portion of the at least one signal carrier/the flex-PCB.

Preferably, the lower contact surface (defining said contact plane) of the sensor head and a lower surface of said cable may be arranged coplanar. In such a design, these surfaces may still be bent into curved shapes, but they do not form a step. In other words, the lower contact surface and the lower surface of said cable may show a continuous transition. Preferably, at least in the area of the transition, the two surfaces may extend in a single (common) plane.

Alternatively, the at least one signal carrier may be formed by at least one light guide. Preferably, each of these light guides (if multiple guides are used) may be coated respectively by a separate cladding. In such a case, said at least one light source and/or said at least one detector can be formed by a respective distal optical element, for example. Such a distal optical element may be, for example, an end facet of a light guide or for example an optical mirror or an optical lens (which may receive light from a light guide and send the light out into tissue). Again, by using (thin) flexible light guides, the course of the middle portion can be easily adapted/flexed during use of the optical sensor.

In the case of using light guides as signal carriers, at least one optoelectronic source of light (e.g., an LED) and at least one optoelectronic detector unit (e.g., a photodiode) may be arranged at a distal end of said cable, either as part of the sensor or as part of a separate electronic module. The light from the optoelectronic source of light may be guided by the at least one light guide to the light source located in the head of the sensor. The latter thus not necessarily produces the light but emits it from the sensor head. For example, the at least one light source located in the sensor head may be functionally coupled to lenses or other optical means for forming a beam of light to be sent out from the head of the sensor. Another one of the at least one light guide may be used to guide light which is received by the detector (in the simplest case an end facet of the respective light guide) located in the sensor head to the optoelectronic detector unit located at the proximal end of the cable.

Most preferably, the least one signal carrier, in particular said printed circuit board, may be supported by or suspended by at least one flexible supporting structure at the location of the smooth curve mentioned before. Most preferable are designs in which the supporting structure is bendable/flexible around a transverse axis (x-axis) of the sensor, because this enables a close and large area skin contact of the sensor.

According to one particular embodiment, the flexible supporting structure may be formed by at least one pillar, which can be provided by the sensor head. It is also possible that the at least one pillar extends from the location of the smooth curve. In this case, the at least one pillar can be embedded in a wall portion of the sensor, for example. When using two or more pillars, the pillars may be separated by gaps from each other. Such a design is beneficial for achieving a high degree of flexibility of the support structure.

The at least one pillar may have a round or rectangular shape, for example; the at least one pillar may also extend longer in a direction perpendicular to the main routing direction (x-direction) of the at least one signal carrier than in said main routing direction (y-direction), which is beneficial for achieving high flexibility for bending around the transverse x-axis

According to one embodiment, the at least one pillar is formed by a lower member of the sensor head, which defines said contact plane.

Alternatively, the at least one pillar may be formed by a molded member. In other words, at least one pillar may be formed separately from said lower member forming part of the sensor head.

One design suggests that a first distal pillar which is provided by the sensor head and which supports said at least one signal carrier (i.e., in particular said flexible printed circuit board) in the middle portion shows a distance D2 from a proximal end of the carrier substrate of D2 ≥ 0.20 mm. Preferably, D2 may additionally such that D2 ≤ 0.60 mm. Such a design results in a compact sensor head and at the same time, efficient underfilling / embedding of the signal carrier / the flex-PCB can be guaranteed, in particular when using vacuum molding.

To improve the optical functionality of the sensor, it is of advantage if the elevation D1 of the carrier substrate from the contact plane is D1 ≥ 1.0 mm. This also results in a bending that can be repeatedly defined accurately.

The mentioned at least one pillar can be a molded member. In this case, it is preferred, if the at least one pillar is molded from a flexible material such as silicone or is formed by an injection molded member. For example, the at least one pillar may be molded into a recess of a wall portion of the sensor.

The sensor head, preferably a lower member of the sensor head, may carry at least two pillars. These at least two pillars may extend to the at least one signal carrier. In particular, the at least two pillars may support the at least one signal carrier (i.e., in particular said flexible printed circuit board) in the middle portion. In such a case, each of said at least two pillars may provide a respective supporting surface which follows the respective smooth curve of the respective middle portion of the at least one signal carrier. As a result, the supporting surfaces may define said smooth, in particular S-shaped, curve. In particular, the respective supporting surface may be curved and hence non-planar.

The lower member of the sensor head mentioned before can be a molded member, for example an injection molded member. Moreover, the lower member may forms said at least two pillars. Hence, the at least two pillars and the lower member may be formed as one piece.

Alternatively, said at least one pillar can be a molded member but can be fabricated separately from said lower member. For example, during molding of the sensor head, the middle portion of the flexible printed circuit board may be temporarily supported by removable metal pillars. After removing these pillars or pins (which may be part of a mold using during the molding step), the resulting voids (at the locations of the pins) below the middle portion of the flex-PCB may be filled up with a casting compound (which may be the same or a different material than that used for molding the sensor head/said lower member) to form molded pillars supporting the middle portion of the flex-PCB in the final sensor device. It is clear, that in this case a boundary may be optically perceivable between the molded member and the molded pillars. These molded pillars may thus form the flexible supporting structure, which supports the smooth curve of the at least one signal carrier, as described above with respect to the optical sensor.

The at least two pillars, in particular said respective supporting surfaces, can also each extend longer, preferably at least 5 times longer, along a transverse axis of the sensor then along a longitudinal axis of the sensor. The longitudinal axis may coincide with a main routing direction of said signal carrier. Such a design provides high robustness to the sensor and at the same time allows sufficient bending in the area of the smooth curve along the transverse axis.

For example, the at least two pillars may be separated from each other along a longitudinal axis of the sensor by interspaces, which are running along a transverse axis of the sensor. Preferably, the transverse axis may extend in a plane that is parallel to a plane defined by the carrier substrate. These interspaces may be air-filled; preferably, however, the interspaces may be filled, at least partly, with a moldable material, in particular a casting compound to increase the robustness of support for the signal carrier. The at least two pillars can also be arranged in a 2-dimensional array with such interspaces running along the transverse axis and along the longitudinal axis of the sensor.

With respect to the smooth curve mentioned before, it is noted that this curve may feature an inflection point indicating a transition of a course of the at least one signal carrier (i.e., in particular of said flexible printed circuit board), from a left-handed bend into a right-handed-bend (or vice versa). In this case, it is preferred for accurate definition of the curve, if the at least one signal carrier, in particular said flexible printed circuit board, is supported by a pillar at the location of the inflection point.

In addition, or alternatively, it is also preferably, if the at least one signal carrier (in particular said flexible printed circuit board), is supported by respective pillars, which are located upstream and downstream of the location of the inflection point, respectively. In other words, from the middle portion onwards the at least one signal carrier, in particular said flexible printed circuit board, may thus rise monotonously above the contact plane to the carrier substrate (w.r.t. to said contact plane).

The sensor head can feature a lower member (which may be, in particular, said lower member mentioned above), which forms a contact surface to be brought into skin contact during a measurement. Preferably, the sensor head design may be such that this contact surface is bendable. In this case, the sensor head can be brought into large-scale skin contact on variously sized skulls (with different curvatures). A center area of said contact surface may coincide with said contact plane mentioned before. The contact surface may also reach into a section of the sensor head corresponding to the middle portion of the at least one signal carrier, such that this section of the sensor head can also be brought into skin contact during a measurement.

In a relaxed state, the contact surface can thus be planar, or it may show a (pre-defined but possibly changeable) curvature around a transverse x-axis of the sensor. In the latter case, it is preferred if the contact surface shows a further curvature along a longitudinal y-axis of the sensor. As will become evident from the Figures, the longitudinal y-axis of the sensor may be defined by the course of the cable, whereas the transverse x-axis may run perpendicular to the longitudinal axis and perpendicular to the z-axis along which the sensor emits and receives light during a measurement.

The carrier substrate may be stiffer than the flexible printed circuit board. For example, it can be attached to two sides of the printed circuit board as a stiffener structure.

The cable may be designed as a flat band. In this case, the flexible printed circuit board may run most preferably in a middle plane of the band.

The cable of the sensor and/or said lower member of the sensor head may be bendable in such a way that a proximal portion of the at least one signal carrier (i.e., in particular of said flexible printed circuit board), can be arranged such that the proximal portion runs parallel to said contact plane; alternatively, the bending may be such that in a relaxed state, the proximal portion may run obliquely and/or curved to said contact plane.

Preferably, the middle portion of the signal carrier / of said flex-PCB can run at an angle α of at least 20° to said contact plane in one portion. Moreover, at least initially on the proximal side, the middle portion may extend parallel to said contact plane in another portion.

The carrier substrate can be supported by supporting members provided by the sensor head, preferably provided by said lower member. In addition, the carrier substrate can be embedded in a moldable material, in particular in said lower member of the sensor head (in case said member is fabricated as a molded member).

The sensor head, in particular said lower member, can be bendable. This may be achieved by form the head / said lower member from a flexible material, preferably from a silicone.

The at least one light source and/or said at least one detector may be encased by at least one metal housing. In this case, the at least one metal housing may be fixed to the carrier substrate, preferably by a solder connection or by gluing. Moreover, the at least one metal housing may be supported by said lower member of the sensor head. Such a design can result in efficient electromagnetic shielding.

For solving the afore-mentioned problem, the invention also suggests a method for fabricating a sensor, in particular according to one of the claims directed towards an sensor or as described herein. In other words, the sensor to be fabricated may comprise: a sensor head bearing at least one light source and at least one detector, which are arranged on a carrier substrate (in particular, respectively). This sensor head may define a contact plane or contact surface to be brought into skin contact during a measurement. The sensor may also comprise a cable featuring at least one signal carrier configured to route signals to and/or from the sensor head.

According to the invention, the method comprises the following steps for solving the problem: a carrier substrate is arranged on a level which is elevated above the contact plane by a distance D1; a middle portion of the at least one signal carrier, which is located between the carrier substrate and a proximal portion of the at least one signal carrier, is bent into an smooth curve; this may be achieved by using temporarily support structures for example, or by using the mentioned at least one pillar directly. Finally, the smooth curve is fixed by embedding the middle portion into a mold material.

This method can be further elaborated, as will now be explained: For example, prior to molding, a pre-fabricated lower member of the sensor may be arranged below the middle portion to define the smooth curve and/or to achieve the desired bending of the middle section.

Alternatively, it is also possible that, during the molding, temporary support structures (which do not form parts of the fabricated sensor later) are used to define the smooth curve temporarily. In this case, the temporary support structures may be removed after the molding to form voids and the voids can then at least partly be filled up with a mold material in a second molding step. This way, a strong support structure for the signal carriers can also be fabricated.

In both cases (using permanent or temporary support structures during molding), the smooth curve may also be maintained in position and shape by clamping from two sides. I.e., two (preferably flexible and permanent or temporary) support structures may be used on both sides of the smooth curve to clamp the middle portion of the at least one signal carrier (in particular of said flexible printed circuit board). If temporary support structures are used, the clamping may be removed after molding, and the remaining voids may be filled up with a molding compound afterwards (at least partly).

Finally, during the molding, a supporting structure (in particular as described in detail before w.r.t. the design of the sensor, i.e., for example a supporting structure formed by at least one pillar) forming part of the fabricated sensor can be used to define the smooth curve.

Preferred examples of the present invention shall now be described in more detail, although the present invention is not limited to these examples: for those skilled in the art, it is obvious that further examples of the present invention may be obtained by combining features of one or more of the patent claims with each other and/or with one or more features of an example as described or illustrated herein.

With reference to the accompanying drawings, where features with corresponding technical function are referenced with same numerals even when these features differ in shape or design:
- Fig. 1: shows a perspective side view on inner components of an optical sensor according to the invention,
- Fig. 2: shows the same optical sensor in a cross-sectional view along a longitudinal y-axis,
- Fig. 3: shows a detail of the cross-sectional side view of Figure 2,
- Fig. 4: shows a lower member of the head of the sensor of Figure 1,
- Fig. 5: shows the lower member of Figure 4 together with a flexible printed circuit board and attached carrier substrate,
- Fig. 6: shows a detail of a cross-sectional side view of another optical sensor according to the invention, in which the middle portion also follows a smooth curve,
- Fig. 7: is a schematic illustration to show the contact plane defined by the head of the sensor and the curvature of the sensor head's contact surface, and
- Fig. 8: shows the optical sensor of Figures 1-3 in a bended state, in which the cable 3 has been bended downwards.

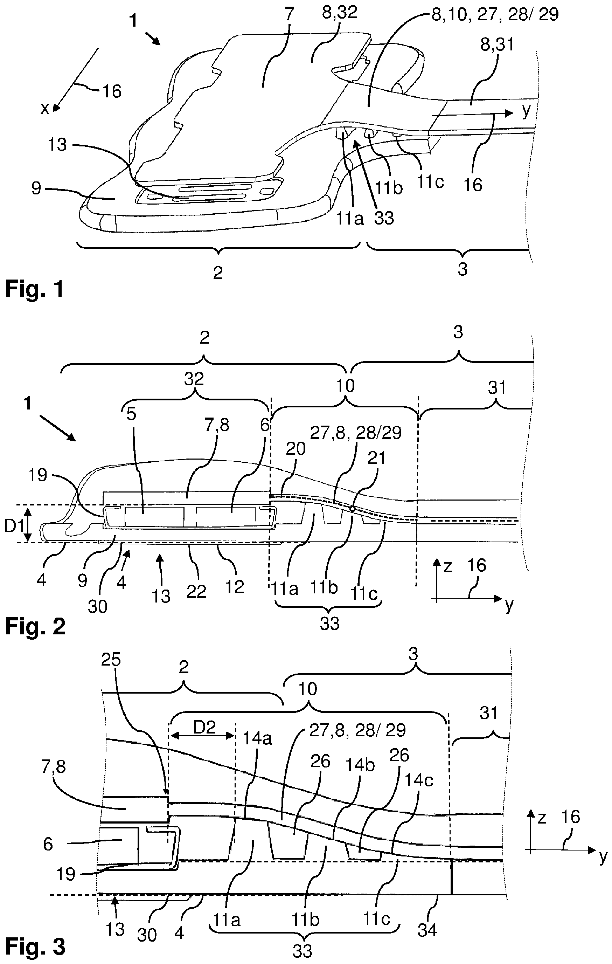

Figure 1 shows a perspective side view on inner components of an optical sensor 1 according to the invention, which is also depicted in Figure 2. The optical sensor 1 is designed as a near infrared spectroscopy (NIRS) sensor and can optically measure oxygenation levels in subcutaneous tissue layers, in particular in cerebral tissue, for vital parameter monitoring. For that purpose, the sensor 1 is brought into skin contact with its lower side, which forms a soft contact surface 12 (cf. Figure 2), on the skull of a patient. IR-light emitted by light sources 5 of the sensor 1 is then irradiated into the tissue (along the z-direction in Figure 2) and the part of the IR-light which is back-scattered by the tissue is collected by the sensor 1 using several detectors 6 located at different source-detector-separations (SDS) from the respective light source 5.

As illustrated in Figure 2, the optical sensor 1 features a sensor head 2 which bears several LEDs as light sources 5 and several photodiodes (PDs) as detectors 6. These optoelectronic components 5,6 are arranged on and electrically connected to a carrier substrate 7 in the form of stiff printed circuit board (PCB) formed from FR4-material and copper conducting lines. The carrier substrate 7 is electrically connected to a second flexible PCB 8, which features several copper conductive tracks as electrical conductors 28 that are running along the longitudinal direction 16 (y-axis) of the sensor 1. Part of the flex-PCB 8 is embedded in a flat cable 3 formed from silicone, with the flex-PCB 8 running in a middle plane of the band formed by the flat cable 3. The conductive tracks of the PCB 8 form signal carriers 27, which serve to route electrical signals to the sensor head 2 to drive and control the operation of the light sources 5 and detectors 6 and to route electrical signals from the sensor head 2 to read-out measurement signals delivered by the detectors 6.

We note at this point that the concept presented herein can also be implemented by replacing one or more of the conductive tracks by light guides 29 as signal carriers 27 (as indicated by the reference numeral 29 in Figures 1 and 2). In this case, an electronic source of light may be used as an external component to deliver optical signals through the light guides to the sensor head 2. In this case, the LEDs may be replaced by light sources which can be formed, for example, simply by end facets of the light guides, to send out light from the sensor 1. In the same manner, other end facets of light guides may be used as detectors to receive the back-scattered light and send these optical signals via the light guide (along the cable) to an electronic detector, which may also be an external component.

As is visible in the side-view of Figure 2, the carrier substrate 7 is elevated above the contact plane 4 (defined by the lower surface of the sensor head 2) by a distance D1 of more than 1 mm. This is necessary to allow beam shaping of the light send out by the LEDs 5. A metal housing 19 is mounted on the carrier substrate 7 to shield the LEDs 5 and PDs 6 from electromagnetic interference. As visible, the carrier substrate 7 rests via the metal housing 19 on a lower member 9 of the sensor head 2, which has been formed by injection molding.

In Figures 1 and 2 it is visible that a middle portion 10 of the flex-PCB 8 (which serves as the signal carrier 27) follows a smooth curve 20. As visible from the more detailed view of Figure 3, this middle portion 10 is located between the carrier substrate 7 (on the left/at the distal end of the flex-PCB 8) and a proximal portion 9 of the flex-PCB 8, which is embedded in the flat cable 3.

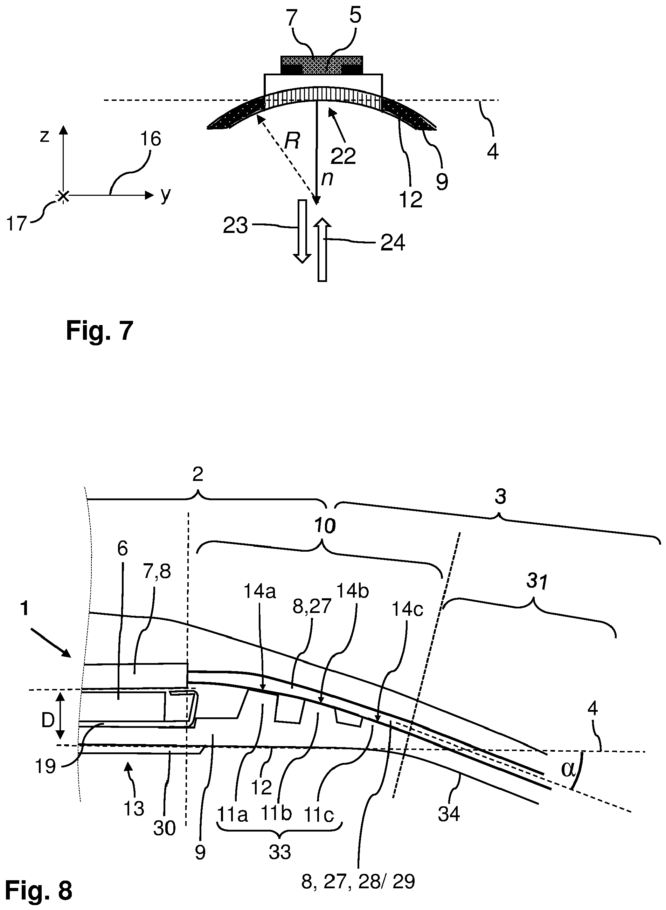

As shown in Figure 1-3, a lower member 9 of the sensor head 2 forms three pillars 11a, 11b and 11c, which form a supporting structure 33 for supporting the flex-PCB 8 at the location of the smooth curve 20 (mainly during manufacturing and prior to molding). As has been mentioned before, the injection-molded and flexible lower member 9 also defines the contact plane 4 that is to be brought into skin contact during a measurement, i.e., it forms a skin interface. More precisely, the lower member 9 forms a bendable contact surface 12 to be brought into skin contact during a measurement and the center area 22 of said surface 12 coincides with the (imaginary) contact plane 4 mentioned before - see for example Figures 2 and 7.

Also note that the middle pillar 11b supports the flex-PCB 8 at the location of an inflection point 21 of the smooth S-shaped curve 20, whereas the distal pillar 11a and the proximal pillar 11c are located upstream and downstream (seen from the perspective of signal routing from the head 2 to the proximal side of the cable 3) from the inflection point 21, respectively. As a result, the signal carrier 27/the flex-PCB 8 rises monotonously above the contact plane 4 to the carrier substrate 7 when following the middle portion 10 onwards to the carrier substrate 7. At the same time, the proximal portion of the flex-PCB 8 just to the right of the middle portion 10 can be bent such that it runs parallel to the contact plane 4, as is illustrated in Figures 2 and 3.

An alternative for achieving a suitable support structure can be to only temporarily support the middle portion 10 of the signal carrier 27 during fabrication of the sensor 1 using, for example, retractable metal pins. This also allows definition of the intended smooth curve 20 of the middle portion, which may then be fixed by embedding the middle portion 10 in a molding compound. Afterwards, the metal pins may be removed, and the resulting voids may then be filled up (at least partly) during a second molding step. In this case, the mold material filled into the voids will form the desired final support structure for the smooth curve 20. In this case, the pillars 11 can thus be molded from a flexible material such as silicone and they can be fabricated separately from the lower member 9.

As Figure 3 shows, the most distal pillar 11a shows a distance D2 from a proximal end 25 of the carrier substrate 7. This distance is chosen to be larger than 0.2 mm and smaller than 0.6 mm. Accordingly, a sufficiently large gap between the distal pillar 11a and the carrier substrate 7 is formed that can be filled with a molding compound. At the same time, the gap is sufficiently small such that the sensor head 2 remains compact and the S-shape curve 20 is accurately controlled.

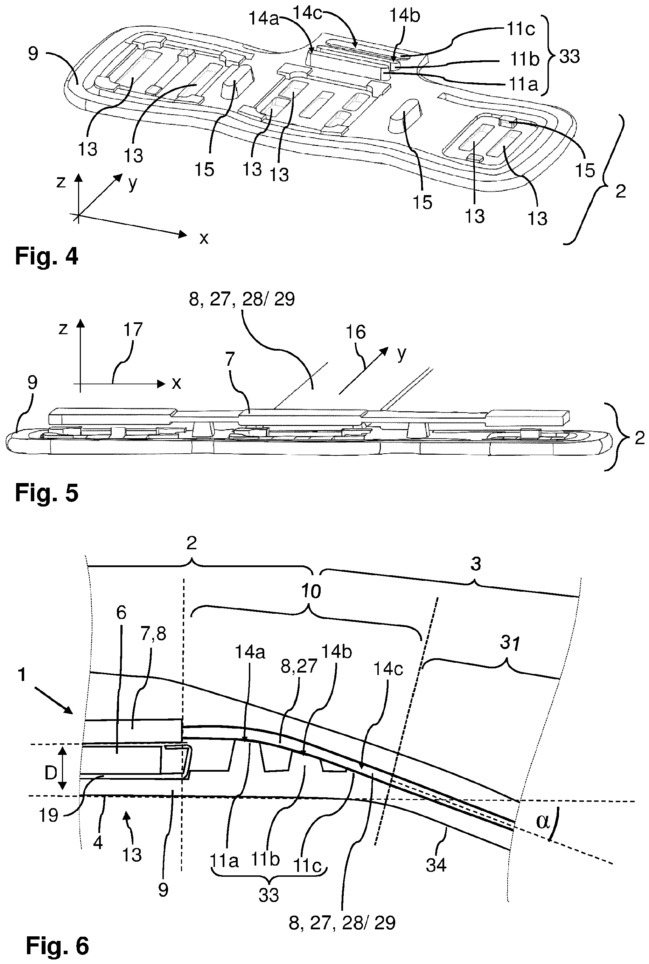

As is revealed by the detailed view of the lower member 9 in Figure 4, the three pillars 11a/11b/11c each extend longer (in fact more than 5x longer) in the transversal x-direction as in the longitudinal y-direction of the sensor. Each pillar 11 forms thus an approximately rectangular supporting surface 14a/14b/14c. These surfaces 14 are non-planar and curved and thereby define the smooth curve 20 of the middle portion 10 of the signal carrier 27 / the flex-PCB 8. It is also visible that the pillars 11 are separated from each other along the longitudinal y-axis 16 by interspaces 26 running along the transverse x-axis 17 (cf. Figure 4 with Figure 1).

As can be seen in Figure 2 and 3, the lower contact surface 12 of the sensor head 2 and a lower surface 34 of the cable 3 are arranged coplanar. Nevertheless, these surfaces 12, 34 may also be bended or designed with a curved shape as shown in figures 6 and 8, but they do not form a step. In other words, the lower contact surface 12 and the lower surface of the cable 3 show a continuous transition, i.e., they merge smoothly into each other without forming a gap or step. Preferably, both surfaces 12, 34 may be formed by a common member as one piece. Also, these two surfaces 12, 34 may extend in a single/common plane, at least in the area of the transition.

In Figure 4, we can also see respective windows 13 (formed by a transparent silicone), which are formed by the lower member 9, whose main body is non-transparent for the NIR-radiation sent out by the light sources 5. Also note the supporting members 15, which serve the purpose of defining the elevation D1 of the carrier substrate 7 above the contact plane 4, as illustrated in Figures 5 and 2.

As shown in figure 4, light sources may be located adjacent to windows 13 on the right side in the drawing, while the detectors may be located adjacent to the windows 13 on the left side and in the middle of the drawing. Of course, other placements of the light sources and or detectors can also be implemented while maintaining the design according to the invention.

Figure 6 shows an alternative design of an optical sensor 1 according to the invention, which also features a signal carrier 17 that is bent into a smooth curve 20 and supported by at least two pillars 11. The design differs, however, from that of Figures 1 to 5 in that - in a relaxed state (i.e., without external stress applied) the proximal portion 31 of the signal carrier 27/the flex-PCB 8 runs obliquely w.r.t. the contact plane 4. In other words, in this design, the middle portion may also be bended into a smooth curve but without external forces applied, the middle portion will run along a smooth curve as shown in Figure 6, which does not resemble an "S". Such a design can help to avoid the formation of pressure marks, when attaching the sensor 1 to the small heads of neonates. It is also possible to form the lower member 9 in such a way that the contact surface 12 can actually be non-planar and show a curvature R, for example along the transverse x-axis 17 as depicted in Figure 7. Also note that the lower member 9 may actually be embedded in an outer soft shell (e.g., a silicone layer) of the sensor head 2. In the same manner, the carrier substrate 7 may also be embedded in a moldable material.

Figure 8 shows the optical sensor 1 of Figures 1-3 in a bended state, in which the cable 3 has been bended downwards such that the curve, which the middle portion 10 follows, now deviates from the initial S-Shape. Due to the flexible nature of the supporting structure 33, which comprises the three pillars 11a, 11b, and 11c (note the deformation in particular of the first pillar 11a), the middle section 10 of the flex-PCB 27 has thus adjusted its smooth course, as can be seen be comparing Figure 8 with Figure 3. As is thus illustrated, during use of the optical sensor 1, the middle section 10 may be flexed and bended easily and as a result, the lower contact surface 12 of the sensor 1 can be brought into conformal skin contact even on highly curved skin surfaces.

In summary, for improving the conformability of a head 2 of an optical sensor 1 and also to improve shielding from ambient light, and for avoiding pressure marks on skin, a novel design of an optical sensor 1 is suggested which features a signal carrier 27, for example in the form of a flexible PCB 8 or in the form of a bundle of light guides 29, which is formed into a smooth continuous curve 20 by a supporting structure 33 that preferably features at least two pillars 11. The support structure, which may still be bendable and allow deformation of the smooth curve 20, helps in guiding the signal carrier 27 in a way that avoids the formation of pressure spots; it also helps to bring a lower contact surface 12 of the sensor's head 2 into close vicinity to the skin during a measurement (cf. Figure 2). Through these measures, more reliable optical measurements can be obtained and the comfort for the patient is also improved.

### List of reference numerals

- 1: sensor
- 2: sensor head (located at the distal end of 1)
- 3: cable (for routing electricals signal from and to 2)
- 4: contact plane (defined by 2 and to be brought in contact with tissue during a measurement)
- 5: light source
- 6: detector
- 7: carrier substrate (carrying 5 and 6)
- 8: flexible printed circuit board (flex-PCB)
- 9: lower member (of 2)
- 10: middle portion (of 8/27)
- 11: pillar (for supporting 8)
- 12: contact surface (of 2 defining 4)
- 13: window (in 11 for transmitting light emitted by 5 or received by 6)
- 14: supporting surface
- 15: supporting member (for supporting 7)
- 16: longitudinal y-axis
- 17: transverse x-axis
- 18: z-axis
- 19: metal housing
- 20: S-curve
- 21: inflection point (of 20 / 8)
- 22: center area (of 12)
- 23: light emission
- 24: light reception
- 25: proximal end (of 7)
- 26: interspace (between 11)
- 27: signal carrier (either an electrical conductor or a light guide, in particular a waveguide)
- 28: electrical conductor
- 29: light guide
- 30: rib (running in y-direction and serving as an optical barrier for mitigating optical cross-talk between 5 and 6)
- 31: proximal portion (of 8/27)
- 32: distal portion (of 8/27)
- 33: supporting structure
- 34: lower surface (of 3)

## Claims

1. **Optical sensor (1),** in particular configured as a NIRS-sensor or as an oximeter, for measuring a physiological parameter in human tissue, the sensor (1) comprising,
- a sensor head (2) bearing at least one light source (5) and at least one detector (6) which are arranged on a carrier substrate (7), wherein the sensor head (2) defines a contact plane (4) to be brought into skin contact during a measurement, and
- a cable (3) comprising at least one signal carrier (27) configured to route signals to and/or from the sensor head (2), **characterized in that,**
- the carrier substrate (7) is elevated above the contact plane (4) by a distance D1.

2. Sensor according to claim 1,
- wherein a middle portion (10) of the at least one signal carrier (27), being located between the carrier substrate (7) and a proximal portion (9) of the at least one signal carrier (27), follows a smooth curve (20), preferably without kinks,
- most preferably wherein the curve (20) shows an S-shape and/or is S-shaped and/or
- wherein the at least one signal carrier (27) is at least one electrical conductor (28), preferably formed by a flexible printed circuit board (8),
- most preferably wherein the carrier substrate (7) is arranged on or formed by the flexible printed circuit board (8),
- in particular wherein said at least one light source (5) and/or said at least one detector (6) are optoelectronic components, which are mounted on the carrier substrate (7),
or
- the at least one signal carrier (27) is at least one light guide (29), preferably coated respectively by a separate cladding,
- in particular wherein said at least one light source (5) and/or said at least one detector (6) is/are formed by a respective distal optical element.

3. Sensor (1) according to claim 1 or 2,
- wherein said at least one signal carrier (27), in particular said printed circuit board (8), is supported by or suspended by at least one flexible supporting structure (33) at the location of the smooth, in particular S-shaped, curve (20),
- preferably wherein the supporting structure (33) is bendable/flexible around a transverse axis (17) of the sensor (1), and/or
- wherein the supporting structure (33) is formed by at least one pillar (11), which is provided by the sensor head (2) or
- wherein at least one pillar (11) extends from the location of the smooth, in particular S-shaped, curve (20), preferably wherein the at least one pillar (11) is embedded in a wall portion of the sensor.

4. Sensor (1) according to any of the preceding claims,
- wherein said at least one pillar (11) is formed
- by a lower member (9) of the sensor head (2) which defines said contact plane (4) or
- by a molded member, in particular separately from a lower member (9) of the sensor head (2).

5. Sensor (1) according to any of the preceding claims,
- wherein a first distal pillar (11a) provided by the sensor head (2) and supporting said at least one signal carrier (27), in particular said flexible printed circuit board (8), in the middle portion (10) shows a distance D2 from a proximal end (25) of the carrier substrate (7) of D2 ≥ 0.20 mm,
- preferably and wherein D2 ≤ 0.60 mm.

6. Sensor (1) according to any of the preceding claims,
- wherein the elevation of the carrier substrate (7) from the contact plane (4) is D1 ≥ 1.0 mm.

7. Sensor (1) according to any of the preceding claims,
- wherein said at least one pillar (11) is a molded member,
- preferably wherein the at least one pillar (11) is molded from a flexible material such as silicone or is formed by an injection molded member.

8. Sensor (1) according to any of the preceding claims,
- wherein the sensor head (2), preferably a lower member (9) of the sensor head (2), carries at least two pillars (11a, 11b, 11c) supporting and/or extending to the at least one signal carrier (27), in particular said flexible printed circuit board (8), in the middle portion (10),
- preferably wherein each of said at least two pillars (11a, 11b, 11c) provides a respective supporting surface (14a, 14b, 14c) which follows the respective smooth curve (20) of the respective middle portion (10) of the at least one signal carrier (27),
- in particular such that the supporting surfaces (14a, 14b, 14c) define said smooth curve (20).

9. Sensor (1) according to the previous claim,
- wherein said lower member (9) is a molded, in particular injection molded, member and forms said at least two pillars (11a, 11b, 11c) or
- wherein said at least one pillar (11) is a molded member and has been fabricated separately from said lower member (9) .

10. Sensor (1) according to claim 8 or claim 9,
- wherein the at least two pillars (11a, 11b, 11c), in particular said respective supporting surfaces (14a, 14b, 14c), each extend longer, preferably at least 5 times longer, along a transverse axis (17) of the sensor (1) then along a longitudinal axis (16) of the sensor (1),
- preferably wherein the transverse axis (17) extends in a plane that is parallel to a plane defined by the carrier substrate (7).

11. Sensor (1) according to any of the claims 8 to 10,
- wherein the at least two pillars (11) are separated from each other along a longitudinal axis (16) of the sensor (1) by interspaces (26) running along a transverse axis (17) of the sensor (1),
- in particular wherein the at least two pillars (11) are arranged in a 2-dimensional array with interspaces (26) running along the transverse axis (17) and along the longitudinal axis (16) of the sensor (1),
- preferably wherein the interspaces (26) are filled, at least partly, with a moldable material, in particular a casting compound.

12. Sensor (1) according to any of the preceding claims,
- wherein the S-shaped curve (20) features an inflection point (21) indicating the transition of the course of the at least one signal carrier (29), in particular of said flexible printed circuit board (8), from a left-handed bend into a right-handed-bend or vice versa,
- preferably wherein the at least one signal carrier (27), in particular said flexible printed circuit board (8), is supported by a pillar (11b) at the location of the inflection point (21) and/or
- preferably wherein the at least one signal carrier (27), in particular said flexible printed circuit board (8), is supported by respective pillars (11a, 11c) which are located upstream and downstream of the location of the inflection point (21), respectively and/or
- wherein, from the middle portion (10) onwards the at least one signal carrier (27), in particular said flexible printed circuit board (8), rises monotonously above the contact plane (4) to the carrier substrate (7).

13. Sensor (1) according to any of the preceding claims,
- wherein the sensor head (2) features a lower member (9), in particular said lower member (9) mentioned above, forming a, preferably bendable, contact surface (12) to be brought into skin contact during a measurement,
- in particular wherein a center area (22) of said contact surface (12) coincides with said contact plane (4) and/or
- wherein said contact surface (12) is
- planar
or
- shows a curvature around a transverse x-axis (17) of the sensor (1),
- preferably and a further curvature along a longitudinal y-axis (16) of the sensor (1).

14. **Method for fabricating a sensor (1),** in particular according to any of the preceding claims, the sensor (1) comprising:
- a sensor head (2) bearing at least one light source (5) and at least one detector (6) which are arranged on a carrier substrate (7), wherein the sensor head (2) defines a contact plane (4) to be brought into skin contact during a measurement, and
- a cable (3) comprising at least one signal carrier (27) configured to route signals to and/or from the sensor head (2), **the method comprising the steps of,**
- arranging a carrier substrate (7) on a level which is elevated above the contact plane (4) by a distance D1,
- bending a middle portion (10) of the at least one signal carrier (27), being located between the carrier substrate (7) and a proximal portion (9) of the at least one signal carrier (27), into a smooth curve (20);
- fixing the smooth curve (20) by embedding the middle portion (10) into a mold material.

15. Method according to the previous claim,
- wherein, prior to molding, a pre-fabricated lower member (9) of the sensor (1) is arranged below the middle portion (10) to define the smooth curve (20),
or
- wherein, during the molding, temporary support structures not forming parts of the fabricated sensor (1) are used to temporarily define the smooth curve (20),
- preferably wherein the temporary support structures are removed after the molding to form voids and the voids are at least partly filled up with a mold material in a second molding step
or
- wherein, during the molding, a supporting structure (33) forming part of the fabricated sensor (1) is used to define the smooth curve (20).
